# EUROPÄISCHE PATENTSCHRIFT

(11) **EP 1 737 787 B1**
(45) Veröffentlichungstag und Bekanntmachung des Hinweises auf die Patenterteilung: **16.08.2017**
(21) Anmeldenummer: 05744560.3
(22) Anmeldetag: 22.04.2005
(51) Int. Cl.: C01B 11/02

(54) **VERFAHREN ZUR ERZEUGUNG VON CHLORDIOXID FÜR DESINFEKTIONSZWECKE UND ZUBEREITUNG DAFÜR**
METHOD FOR THE PRODUCTION OF CHLORINE DIOXIDE FOR DISINFECTION PURPOSES AND PREPARATION THEREFOR
PROCEDE DE PRODUCTION DE DIOXYDE DE CHLORE UTILISE A DES FINS DE DESINFECTION ET PREPARATION APPROPRIEE

(30) Priorität: 22.04.2004 DE 102004020815
(43) Veröffentlichungstag der Anmeldung: 03.01.2007
(73) Patentinhaber: BWT Aktiengesellschaft, 5310 Mondsee (AT)
(72) Erfinder: BERGMANN, Ralph, 69469 Weinheim (DE); JOHANN, Jürgen, 5310 Mondsee (AT)
(74) Vertreter: Pfiz, Thomas
(86) Internationale Anmeldenummer: PCT/EP2005/004315
(87) Internationale Veröffentlichungsnummer: WO 2005/102920

(56) Entgegenhaltungen:
- EP-A- 0 832 845
- WO-A-03/097526
- US-A- 2 482 891
- US-A- 3 585 147
- US-A- 4 874 489
- US-A- 5 885 543
- US-A1- 2003 080 317
- "BWT Sanitabs Broschüre", 2017, BWT

## Beschreibung

Die Erfindung betrifft ein Verfahren zur Erzeugung von Chlordioxid für Desinfektionszwecke, bei dem ein Zweikomponentensystem in wässriger Lösung in Ionenaustauschern eingesetzt wird. Die Erfindung betrifft weiter eine Zubereitung für ein solches Verfahren. US4874489 offenbart ein Verfahren zur Herstellung von Chlordioxid worin eine wässrige Lösung von Natriumchlorit in Anwesenheit von Natriumchlorid und unter Anwendung von UV Strahlung reagieren. Die auf dem Markt befindlichen Zweikomponentensysteme flüssig/fest und flüssig/flüssig zur Herstellung von Chlordioxid vor Ort arbeiten mit einer Säure (speziell Salzsäure) und Natriumchlorit oder mit einem Oxidationsmittel (z.B. einem Peroxodisulfat) und Natriumchlorit, ggf. auch mit einem immobilisierten Katalysator, z.B. Platinoxid. Nach der Mischung der beiden Komponenten wird eine chlordioxidhaltige Lösung gebildet. Der Umgang mit Salzsäure ist jedoch aufgrund der Neigung zum Ausgasen problematisch. Durch die Verwendung von oxidativ und/oder sauer wirkenden Salzen wird die Kinetik der Chlordioxidbildung derart beschleunigt, dass es innerhalb kurzer Zeit zu einem nahezu vollständigen Umsatz kommt. Als Konsequenz müssen die Ausgangsstoffe immer getrennt gelagert werden. Nach Abschluss der Bildungsreaktion nimmt aber auch die Geschwindigkeit des Zerfallprozesses zu. Die angesetzten Lösungen sind daher nur begrenzt haltbar. Ein weiterer Nachteil der hohen Reaktionseffizienz liegt darin, dass immer in Verdünnung gearbeitet werden muss, um eine Gefährdung durch gebildetes Chlordioxid auszuschließen. Lösungen ab ca. 8 g/l Chlordioxid mit überstehendern Gaspolster können explodieren.

Allen genannten Prozessen liegt die Oxidation des Chloritanions zu Chlordioxid zugrunde. Die Oxidation wird entweder durch starke Oxidationsmittel (elektrolytischer Strom, Peroxodisulfat oder Chlor) oder durch den Säurezusatz oder durch die Verwendung eines immobilisierten Katalysators beschleunigt. Wird keine dritte Komponente als Stabilisierungsmittel eingesetzt, so werden die Ausgangsstoffe in verschiedenen Gebinden verpackt, um eine vorzeitige Reaktion zu verhindern, oder es wird eine inerte Trennschicht zwischen den beiden Reaktionspartnern eingebracht.

Während die genannten Verfahren unter dem Aspekt des maximierten chemischen Umsatzes und damit der Prozessökonomie stehen, ist dies bei der Anwendung von Chlordioxid zur Desinfektion von Ionenaustauschern oder auch in Lebensmitteln und Kosmetika zweitrangig. Hier gilt es, die entsprechenden Chemikalienrechtsverordnungen einzuhalten.

Bei der Regeneration von Ionenaustauschern, die u. a. der Enthärtung von Wasser dienen, werden zumeist Natrium- oder Kaliumchlorid, teilweise auch Magnesiumchlorid eingesetzt. Beim Enthärtungsprozess werden Calciumionen und Magnesiumionen gegen Natriumionen oder Kaliumionen ausgetauscht, teilweise auch nur Calciumionen gegen Magnesiumionen. Ein Problem von Ionenaustauschern ist deren Neigung zu verkeimen. Um eine Verkeimung zu verhindern existieren diverse Desinfektionsverfahren, die jedoch als separater Schritt im Betrieb des Ionenaustauschers durchgeführt werden müssen.

Ausgehend hiervon liegt der Erfindung die Aufgabe zugrunde, die im Stand der Technik aufgetretenen Nachteile zu vermeiden und mit einem einfach handhabbaren System eine desinfizierende Wirkung insbesondere in Geräten der Wasseraufbereitung direkt am Einsatzort zu erreichen.

Zur Lösung dieser Aufgabe wird die im Patentanspruch 1 bzw. 9 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, Chlordioxid als biocide Substanz direkt am Einsatzort bereitzustellen, ohne dass die Ausgangszubereitung ein erhöhtes Gefahrenpotential besitzt. Dementsprechend wird in verfahrensmäßiger Hinsicht vorgeschlagen, dass das Zweikomponentensystem aus einer Chloritkomponente und einer Salzkomponente gebildet wird, wobei die Chloritkomponente ein Erdalkali- und/oder Alkalichlorit enthält, und wobei die Salzkomponente ein Alkali- oder Erdalkalimetall-Halogenid oder -Sulfat oder - Nitrat oder eine Mischung davon als Hauptbestandteil (Hauptbestandteil im Sinne eines Anteils von mindestens 98 Gew.%) enthält, und dass die Chloritkomponente und die Salzkomponente in wässriger Lösung gemischt und dadurch zur Reaktion gebracht werden, wobei ein technisches Regeneriersalz für Ionenaustauscher als Salzkomponente verwendet wird und die Salzkomponente in wässriger Lösung weder sauer noch oxidativ noch katalytisch bezüglich der Umsetzung des Chlorits wirkt.

Der Vorteil dieses Zweikomponentensystems besteht in seiner guten Handhabbarkeit und Einfachheit sowohl hinsichtlich der minimierten Gefährdung durch die eingesetzten Chemikalien (keine Chemikalien, die als Lösungen zu Verätzungen führen) als auch auf deren Verfügbarkeit und Beschaffungskosten. Überraschenderweise wurde festgestellt, dass die Oxidation des Chloritanions auch ohne zusätzliche Oxidationsmittel oder Sauren oder Katalysatoren in dem für die obigen Anwendungen beschriebenen Rahmen günstig abläuft. Durch die Verwendung von nicht oxidativ bzw. sauer oder katalytisch bezüglich der Oxidation des Chlorits wirkenden Salzen wird die Kinetik der Chlordioxidbildung in vorteilhafter Weise verlangsamt, so dass es nicht innerhalb kürzester Zeit zu einem nahezu 100 %igen Umsatz kommt. Indem ein technisches Regeneriersalz für Ionenaustauscher z.B. nach der Norm EN 973 als Salzkomponente verwendet wird, bietet sich die Möglichkeit, ein Regeneriersalz zu fertigen, das eine biocide Komponente enthält. Dadurch entfallen aufwendige Desinfektionsschritte und apparative Einrichtungen zur Durchführung von Desinfektionen in der Anwendung des Regerteriersalzes z.B. in Ionenaustauschern zur Wasserenthärtung

Vorteilhafterweise werden die Chloritkomponente und die Salzkomponente als insbesondere homogenes binäres Feststoffgemisch oder als Formling in Wasser gelöst. Dadurch bietet sich die Möglichkeit, ein kombiniertes Desinfektions- und Regeneriersalz bereitzustellen. Das Chlordioxid bildet sich erst beim Auflösen in Wasser.

Eine alternative Ausführung sieht vor, dass die Chloritkomponente als vorgefertigte wässrige Lösung zu der festen Salzkomponente hinzugefügt wird. Das Chlordioxid bildet sich durch die teilweise schon in den Ausgangssalzen enthaltene Feuchtigkeit und den Wasseranteil der Chloritlösung.

Sofern ausschließlich mit Lösungen gearbeitet werden soll, können eine Lösung der Chloritkomponente und eine Lösung der Salzkomponente gemischt werden.

Besonders vorteilhaft ist es, wenn eine Salzkomponente eingesetzt wird, die in wässriger Lösung im Wesentlichen neutral oder alkalisch reagiert. Bevorzugt besteht die Salzkomponente aus Natriumchlorid.

Die Salzkomponente kann zusätzlich Nebenbestandteile wie Fertigungshilfsstoffe oder Verunreinigungen mit einem Anteil von max. 2 Gew% enthalten.

Vorteilhafterweise besteht die Chloritkomponente aus Natriumchlorit. Bei einem Gesamtgehalt von Natriumchlorit kleiner 3 Gew.%, bezüglich des Gesamtsystems ist eine Natriumchlorid-Natriumchlorit-Zubereitung auch nicht kennzeichnungspflichtig im Sinne des Chemikaliengesetzes(EG-Richtlinie 1999/45 EG), was die Akzeptanz deutlich erhöht.

Die Erfindung umfasst auch eine Zubereitung zur Erzeugung von Chlordioxid in wässriger Lösung für Desinfektionszwecke in Ionenaustauschern, wobei die Zubereitung ein Zweikomponentensystem enthält, welches aus einer Chloritkomponente und einer Salzkomponente gebildet ist, wobei die Chloritkomponente ein Erdalkali- und/oder Alkalichlorit enthält und wobei die Salzkomponente ein Natrium- oder Kalium-Halogenid oder -Sulfat oder -Nitrat oder eine Mischung davon als Hauptbestandteil mit einem Anteil von mindestens 98 Gew.% enthält, und wobei die Salzkomponente durch ein technisches Regeneriersalz für Ionenaustauscher gebildet ist und die chemische Eigenschaft aufweist, dass sie in wässriger Lösung gegenüber Chlorit weder als Säure noch als Oxidationsmittel noch als Katalysator wirkt.

Im Folgenden wird die Erfindung anhand von Ausführungsbeispielen näher erläutert.

Es wurden vier homogene Zweikomponentenmischungen hergestellt bestehend aus jeweils 26g Salzkomponente (Natriumchlorid (Qualität p.a.), Kaliumchlorid (Qualität p.a.), Magnesiumsulfatheptahydrat (Qualität p.a.), Regeneriersalz gemäß EN 973) und jeweils 0,75 g Natriumchlorit (Qualität techn.) als Chloritkomponente. Die Mischung mit Magnesiumsulfatheptahydrat diente als Vergleich.

Den vier homogenen Zweikomponenten-Feststoffmischungen wurden jeweils 75 ml vollentsalztes Wasser zugesetzt. Nach 15 Minuten wurde der pH-Wert der flüssigen Phase und nach 120 Minuten Reaktionszeit die Konzentration an Chlordioxid bestimmt. Die Ergebnisse waren wie folgt:

| | | |
|---|---|---|
| Natriumchlorid, p.a. | pH= 9,58 | 1,38 Mg/l ClO₂ |
| Kaliumchlorid, p.a. | pH= 10,35 | 30,5 mg/l ClO₂ |
| Magnesiumsulfat, p.a. | pH= 8,50 | 1,86 Mg/l ClO₂ |
| Natriumchlorid (Reg-Salz) | pH=9,40 | 39,2 mg/l ClO₂ |

Für die Zubereitung eines binären homogenen Feststoffgemischs wurde eine Verreibung hergestellt aus 0,01% bis 3% technischem Natriumchlorit und 99,9 bis 97% Regeniersalz für Ionenaustauscher (NaCl).

Anschließend wurde so viel Wasser zu der Verreibung gegeben, dass ein Bodenkörper aus Feststoff bestehen blieb und sich eine gesättigte Salzsole bildete. Innerhalb eines Zeitraumes von mindestens 15 Minuten begann sich Chlordioxid zu bilden. Die Chlordioxidbildung war daran zu erkennen, dass sich die anfänglich farblose Lösung gelbgrün einfärbte. Wurde der Gehalt an Natriumchlorit erhöht, so erhöhte sich die Ausbeute an Chlordioxid.

Die gebildete Chlordioxidmenge war schon nach 15 Minuten um den Faktor 4 höher als das in einer ausschließlich aus technischem Natriumchlorit bestehenden Lösung gebildete Chlordioxid. In Abhängigkeit von den Umgebungsbedingungen kam die Chlordioxidbildung nach einiger Zeit (einige Stunden) zum Erliegen.

Zur vereinfachten Dosierung wurde bei 500 bar Pressdruck eine Tablette aus einer homogenen Mischung von 20 g gemahlenem Spülmaschinen-Regeneriersalz (Fa. Henkel, Somat) und 0,2 g NaClO₂, technisch (Fa. Riedel de Haen) gepresst. Die Auflösung dieser Tablette in 800 ml Wasser der öffentlichen Wasserversorgung ergab 28 mg/l ClO₂.

Die theoretische Umsatzberechnung ergibt folgende Werte:
Technische Ware enthält nur 80% an NaClO2, also vorliegend 0,16 g entsprechend 1,78 mmol. Daraus können gemäß der folgenden hypothetischen Reaktion

   5 ClO₂⁻ + 2 H₂O -> Cl⁻ + 4 ClO₂ + 4 OH⁻

   1,78*4/5 mmol ClO₂ gebildet werden. Dies entspricht 95,3 mg (molare Masse ClO₂ : 67g). Daraus ergibt sich ein Umsatz von ca. 24%.

Die Reaktionsgeschwindigkeit und die Lage des Gleichgewichts der Chlordioxidbildung ist stark pH-Wert abhängig. In sauren Lösungen würde eine schnelle nahezu 100%-ige Umsetzung erfolgen. Je höher der Lösungs-pH-Wert, desto langsamer verläuft die Chlordioxidbildung und desto geringer ist der Umsatz. Vorteilhaft ist die Tatsache, dass die Chlorid-Anionen katalysierend wirken. Diese sind in ausreichender Konzentration vorhanden.

Die Bespiele zeigen, dass trotz des alkalischen pH-Werts der Ausgangskomponenten und deren nicht oxidativer Wirkung und ohne den Zusatz einer katalytisch wirkenden Komponente bzw. eines Katalysators eine signifikante und für Desinfektionszwecke ausreichende Chlordioxidbildung auftritt.

## Patentansprüche

1. Verfahren zur Erzeugung von Chlordioxid für Desinfektionszwecke in wässriger Lösung in Ionenaustauschern, insbesondere in der Wasseraufbereitung oder in Haushaltsgeräten, insbesondere Geschirrspülern, **dadurch gekennzeichnet, dass** ein Zweikomponentensystem eingesetzt wird, welches aus einer Chloritkomponente und einer Salzkomponente gebildet wird, wobei die Chloritkomponente ein Erdalkali- und/oder Alkalichlorid enthält und wobei die Salzkomponente ein Natrium- oder Kalium-Halogenid oder -Sulfat oder -Nitrat oder eine Mischung davon als Hauptbestandteil enthält, und dass die Chloritkomponente und die Salzkomponente in wässriger Lösung gemischt und dadurch zur Reaktion gebracht werden, wobei ein technisches Regeneriersalz für Ionenaustauscher als Salzkomponente verwendet wird und die Salzkomponente gegenüber Chlorit weder als Säure noch als Oxidationsmittel noch als Katalysator wirkt.

2. Verfahren nach Anspruch 1, **dadurch gekennzeichnet, dass** die Chloritkomponente und die Salzkomponente als insbesondere homogenes binäres Feststoffgemisch oder als Formling in Wasser gelöst werden.

3. Verfahren nach Anspruch 1, **dadurch gekennzeichnet, dass** die Chloritkomponente als vorgefertigte wässrige Lösung zu der festen Salzkomponente hinzugefügt wird.

4. Verfahren nach Anspruch 1, **dadurch gekennzeichnet, dass** eine Lösung der Chloritkomponente und eine Lösung der Salzkomponente gemischt werden.

5. Verfahren nach einem der Ansprüche 1 bis 4, **dadurch gekennzeichnet, dass** die Salzkomponente in wässriger Lösung im Wesentlichen neutral oder alkalisch reagiert.

6. Verfahren nach einem der Ansprüche 1 bis 5, **dadurch gekennzeichnet, dass** die Salzkomponente aus Natriumchlorid besteht.

7. Verfahren nach einem der Ansprüche 1 bis 6, **dadurch gekennzeichnet, dass** die Salzkomponente zusätzlich Nebenbestandteile wie Fertigungshilfsstoffe oder Verunreinigungen mit einem Anteil von max. 2 Gew.% enthält.

8. Verfahren nach einem der Ansprüche 1 bis 7, **dadurch gekennzeichnet, dass** die Chloritkomponente aus Natriumchlorit vorzugsweise mit einem Gesamtgehalt kleiner 3 Gew.% besteht.

9. Zubereitung zur Erzeugung von Chlordioxid für Desinfektionszwecke in wässriger Lösung in Ionenaustauschern, insbesondere in der Wasseraufbereitung oder in Haushaltsgeräten, insbesondere Geschirrspülern, **dadurch gekennzeichnet, dass** die Zubereitung ein Zweikomponentensystem enthält, welches aus einer Chloritkomponente und einer Salzkomponente gebildet ist, wobei die Chloritkomponente ein Erdalkali- und/oder Alkalichlorit enthält und wobei die Salzkomponente ein Natrium- oder Kalium-Halogenid oder -Sulfat oder -Nitrat oder eine Mischung davon als Hauptbestandteil mit einem Anteil von mindestens 98 Gew.% enthält, und dass die Salzkomponente durch ein technisches Regeneriersalz für Ionenaustauscher gebildet ist und die chemische Eigenschaft aufweist, dass sie in wässriger Lösung gegenüber Chlorit weder als Säure noch als Oxidationsmittel noch als Katalysator wirkt.

10. Zubereitung nach Anspruch 9 als Formling, insbesondere als Mantel- oder Schichttablette.

11. Zubereitung nach Anspruch 9 als vorzugsweise homogenes binäres Feststoffgemisch.

12. Zubereitung nach einem der Ansprüche 9 bis 11, **dadurch gekennzeichnet, dass** die Salzkomponente aus Natriumchlorid besteht.

13. Zubereitung nach einem der Ansprüche 9 bis 12, **dadurch gekennzeichnet, dass** die Salzkomponente zusätzlich Nebenbestandteile wie Fertigungshilfsstoffe oder Verunreinigungen mit einem Anteil von max. 2 Gew.% enthält.

14. Zubereitung nach einem der Ansprüche 9 bis 13, **dadurch gekennzeichnet, dass** die Chloritkomponente aus Natriumchlorit besteht.

## Claims

1. Method for the production of chlorine dioxide for disinfection purposes in aqueous solution, in ion exchangers, particularly in water treatment or in household appliances, particularly dishwashers, **characterized in that** a two component system is used which is formed from a chlorite component and a salt component, wherein the chlorite component contains an earth alkali chlorite and/or alkali chlorite and wherein the salt component contains a sodium-halogenide or potassium-halogenide or a -sulfate or -nitrate or a mixture thereof, as the main component, and that the chlorite component and the salt component are mixed in aqueous solution and thereby are brought to a reaction, wherein a technical regeneration salt for ion exchangers is used as the salt component and the salt component acts against chlorite neither as acid nor as oxidant nor as catalyst.

2. Method according to claim 1, **characterized in that** the chlorite component and the salt component are dissolved in water, particularly as a homogeneous binary solid substance mixture or as a shaped product.

3. Method according to claim 1, **characterized in that** the chlorite component is added to the solid salt component as a prefinished aqueous solution.

4. Method according to claim 1, **characterized in that** a solution of the chlorite component and a solution of the salt component are mixed.

5. Method according to one of claims 1 to 4, **characterized in that** the salt component reacts essentially in neutral or alkaline manner in aqueous solution.

6. Method according to one of claims 1 to 5, **characterized in that** the salt component consists of sodium chloride.

7. Method according to one of claims 1 to 6, **characterized in that** the salt component additionally contains secondary components such as production aids or contaminants, with a proportion of max. 2 wt.-%.

8. Method according to one of claims 1 to 8, **characterized in that** the chlorite component consists of sodium chlorite, preferably with a total content of less than 3 wt. %.

9. Preparation for the production of chlorine dioxide for disinfection purposes in aqueous solution, in ion exchangers, particularly in water treatment or in household appliances, particularly dishwashers, **characterized in that** the preparation contains a two component system, which is formed from a chlorite component and a salt component, wherein the chlorite component contains an earth alkali chlorite and/or alkali chlorite, and wherein the salt component contains a sodium-halogenide or potassium-halogenide or a -sulfate or -nitrate or a mixture thereof, as the main component with a proportion of at least 98 wt.-%, and that the salt component is formed by a a technical regeneration salt for ion exchangers and has the chemical characteristic that in aqueous solution it acts against chlorite neither as acid nor as oxidant nor as catalyst.

10. Preparation according to claim 9 as a shaped product, particularly as a mantled or layered tablet.

11. Preparation according to claim 9 as a preferably homogeneous binary solid substance mixture.

12. Preparation according to one of claims 9 to 11, **characterized in that** the salt component consists of sodium chloride.

13. Preparation according to one of claims 9 to 12 **characterized in that** the salt component additionally contains secondary components such as production aids or contaminants, with a proportion of max. 2 wt.-%.

14. Preparation according to one of claims 9 to 13, **characterized in that** the chlorite component consists of sodium chlorite.

## Revendications

1. Procédé pour la production de dioxyde de chlore à des fins de désinfection en solution aqueuse dans des échangeurs d'ions, en particulier dans la préparation de l'eau ou dans des appareils domestiques, en particulier dans des lave-vaisselle, **caractérisé en ce qu'**on utilise un système à deux composants, qui est formé par un composant de type chlorite et un composant de type sel, le composant de type chlorite contenant un chlorite de métal alcalino-terreux et/ou de métal alcalin et le composant de type sel contenant un halogénure, un sulfate ou un nitrate de sodium ou de potassium ou un mélange de ceux-ci comme constituant principal et **en ce que** le composant de type chlorite et le composant de type sel sont mélangés en solution aqueuse et ainsi amenés à réagir, un sel de régénération technique pour échangeur d'ions étant utilisé comme composant de type sel et le composant de type sel n'agissant, ni comme acide, ni comme oxydant, ni comme catalyseur par rapport au chlorite.

2. Procédé selon la revendication 1, **caractérisé en ce que** le composant de type chlorite et le composant de type sel sont dissous dans l'eau sous forme de mélange solide binaire en particulier homogène ou sous forme de pièce façonnée.

3. Procédé selon la revendication 1, **caractérisé en ce que** le composant de type chlorite est ajouté sous forme de solution aqueuse préparée au préalable au composant de type sel solide.

4. Procédé selon la revendication 1, **caractérisé en ce qu'**une solution du composant de type chlorite et une solution du composant de type sel sont mélangées.

5. Procédé selon l'une quelconque des revendications 1 à 4, **caractérisé en ce que** le composant de type sel réagit en solution aqueuse de manière essentiellement neutre ou alcaline.

6. Procédé selon l'une quelconque des revendications 1 à 5, **caractérisé en ce que** le composant de type sel est constitué par du chlorure de sodium.

7. Procédé selon l'une quelconque des revendications 1 à 6, **caractérisé en ce que** le composant de type sel contient en plus des constituants secondaires, tels que des adjuvants de fabrication ou des impuretés en une proportion d'au maximum 2% en poids.

8. Procédé selon l'une quelconque des revendications 1 à 7, **caractérisé en ce que** le composant de type chlorite est constitué par du chlorite de sodium, de préférence à une teneur totale inférieure à 3% en poids.

9. Composition pour la production de dioxyde de chlore à des fins de désinfection en solution aqueuse dans des échangeurs d'ions, en particulier dans la préparation de l'eau ou dans des appareils domestiques, en particulier dans des lave-vaisselle, **caractérisé en ce que** la composition contient un système à deux composants, qui est formé par un composant de type chlorite et un composant de type sel, le composant de type chlorite contenant un chlorite de métal alcalino-terreux et/ou de métal alcalin et le composant de type sel contenant un halogénure, un sulfate ou un nitrate de sodium ou de potassium ou un mélange de ceux-ci comme constituant principal à une proportion d'au moins 98% en poids et **en ce que** le composant de type sel est formé par un sel de régénération technique pour échangeur d'ions et présente la propriété chimique de ne pas agir, ni comme acide, ni comme oxydant, ni comme catalyseur par rapport au chlorite en solution aqueuse.

10. Composition selon la revendication 9 sous forme de pièce façonnée, en particulier sous forme de comprimé enrobé ou à couches.

11. Composition selon la revendication 9 sous forme de mélange solide binaire de préférence homogène.

12. Composition selon l'une quelconque des revendications 9 à 11, **caractérisée en ce que** le composant de type sel est constitué par du chlorure de sodium.

13. Composition selon l'une quelconque des revendications 9 à 12, **caractérisée en ce que** le composant de type sel contient en plus des constituants secondaires, tels que des adjuvants de fabrication ou des impuretés à une proportion d'au maximum 2% en poids.

14. Composition selon l'une quelconque des revendications 9 à 13, **caractérisée en ce que** le composant de type chlorite est constitué par du chlorite de sodium.
